# EUROPEAN PATENT SPECIFICATION

(11) **EP 4 480 438 B1**
(45) Date of publication and mention of the grant of the patent: **31.12.2025**
(21) Application number: 23180991.4
(22) Date of filing: 22.06.2023
(51) Int. Cl.: A61B 34/20, A61B 17/16, A61B 90/00

(54) **TECHNIQUE FOR DETERMINING AN ANGULAR ORIENTATION BETWEEN A NON-ROTATIONALLY SYMMETRIC WORKING END OF A TOOL AND A TOOL TRACKER**
TECHNIK ZUR BESTIMMUNG EINER WINKELLAGE ZWISCHEN EINEM NICHT ROTATIONSSYMMETRISCHEN ARBEITSENDE EINES WERKZEUGS UND EINEM WERKZEUGTRACKER
TECHNIQUE DE DÉTERMINATION D'UNE ORIENTATION ANGULAIRE ENTRE UNE EXTRÉMITÉ DE TRAVAIL NON SYMÉTRIQUE EN ROTATION D'UN OUTIL ET UN DISPOSITIF DE SUIVI D'OUTIL

(43) Date of publication of application: 25.12.2024
(73) Proprietor: Stryker European Operations Limited, Carrigtwohill, Co. Cork T45 HX08 (IE)
(72) Inventor: GERST, Maximilian, 79098 Freiburg im Breisgau (DE); GRAS, Guillaume, 2502 Bienne (CH)
(74) Representative: Röthinger, Rainer

(56) References cited:
- EP-A1- 1 769 768
- US-A1- 2007 016 009
- US-A1- 2008 051 768
- US-A1- 2018 107 291

## Description

### Technical Field

The present disclosure generally relates to a tool having a first tool part configured to be fixed to a second tool part in one of multiple angular orientations. The first tool part has a non-rotationally symmetric working end and the second tool part carries a tool tracker. In particular, a method, a computer program product, an apparatus, a tool and a system for determining an angular orientation between the non-rotationally symmetric working end and the tool tracker are presented.

### Background

In surgical procedures, the surgeons are regularly assisted by various trackable tools and surgical systems for tracking and navigating the tools. The tools have working ends (e.g., to manipulate tissue, to attach a tracker to bone, etc.) the positions of which have to be determined with high precision, for example relative to a patient anatomy. In case of non-rotationally symmetric working ends, also the orientations of the working ends need to be inferred.

For determining the position and orientation of a tool's working end in a surgical procedure, the tool has first to be calibrated. In particular, the position and orientation of the working end of the tool relative to a tool tracker attached thereto has to be determined prior to the actual surgical intervention. The position and orientation of the working end relative to a patient anatomy may then be calculated based on the calibration and tracking data indicative of the position and orientation of the tool tracker.

For calibrating a tool, an additional trackable calibration device may be utilized. The tool's working end is brought in contact with a predefined portion (e.g., a divot) of the calibration device to determine the position of the working end relative to the tool tracker. However, such a calibration fails to provide the angular orientation of the working end relative to the tool tracker and, thus, is only reliable for tools having a rotationally symmetric working end, such as a pointed tip.

An alternative technique for calibrating a tracked surgical tool is described in EP 1 769 768 A1. To enable calibration of the tool without an additional calibration device, a calibration reference point is located on the tool. The calibration reference point has a predetermined position to the tool's working end and is configured to be brought in engagement with another tracked tool so that for calibration purposes, the position of the calibration reference point can be determined from the tracked position of the other tool. However, the angular orientation of the working end relative to a tool tracker is not considered.

Document US 2007/016009 A1 disclose an array for use with a surgical navigation system. The array comprises a frame and first, second and third markers attached to the frame. The first, second and third markers are detectable by a tracking system used in image guided surgery, and the first marker is movable relative to the frame. At least one marker may slide along the frame from a first position where it is held in place to a second position where it is also held in place. One or more of the markers may be removed from the frame at a first position and reattached to the frame at a second position. A portion of the frame itself may move with the moveable marker.

US 20018/0107291 A1 discloses a system and a method for digitizing locations within a coordinate system. A device may include a sleeve including a sleeve tracking marker and a tracked probe portion including an array of tracking markers and a probe tip. Movement of the probe tip relative to the sleeve between at least a first position and a second position may be monitored by tracking the sleeve tracking marker relative to at least one tracking marker of the array of tracking markers.

US 2008/0051768 A1 discloses a medical instrument. The medical instrument has a handle and a functional portion. The medical instrument includes a first part, a second part selectively couplable to the first part and a plurality of trackable markers arranged on the instrument. At least one marker is arranged on the first part and at least one marker is arranged on the second part

Since the calibration techniques discussed above fail to provide an angular orientation of a non-rotationally symmetric working end of a tool relative to the tool tracker, the calibration techniques are not suitable for reliably calibrating such a tool. For example, knowing the correct angular orientation of a flat tool blade is evidently crucial to precisely generate instructions for navigating the blade relative to a patient anatomy.

If the non-rotationally symmetric working end of a tool has a predefined angular and positional relationship to the tool tracker, this relationship can be easily be pre-configured for the navigation algorithm (e.g., pre-programmed or loaded from a tool memory). However, there also exist tools having a first tool part with the working end, that are configured to be fixed in multiple angular orientations to a second tool part carrying the tool tracker. For example, the second tool part may be a handle to be used with various exchangeable first tool parts having different working ends. Such tools may be subject to a difference between an expected and an actual angular orientation of the non-rotationally symmetric working end relative to the handle and, thus, relative to the tool tracker, giving rise to a so-called clocking error. The clocking error may result from manufacturing tolerances, wear of material or a varying force (e.g., torque) applied when attaching the first tool part to the second tool part, and may lead to erroneous tool calibration and associated health risks.

### Summary

There is a need for a technique of determining an angular orientation between a non-rotationally symmetric working end of a first tool part and a tool tracker carried by a second part of the tool, wherein the first tool part is configured to be fixed to the second tool part in one of multiple angular orientations.

According to a first aspect, a method for determining an angular orientation between a non-rotationally symmetric working end of a first part of a tool and a tool tracker carried by a second part of the tool is provided. The first tool part is configured to be fixed to the second tool part in one of multiple angular orientations and comprises a reference structure. The reference structure is traceable by a tracked pointer and has a predefined angular relation to the working end. The method comprises receiving first position data indicative of one or more positions of the tool tracker, receiving second position data indicative of pointer positions while the pointer is tracing the reference structure, and determining, based on the received first and second position data and the predefined angular relation between the reference structure and the working end, the angular orientation of the working end relative to the tool tracker.

The trackers described herein may be trackers commonly used for tracking and/or navigation purposes. For example, the trackers may comprise optical trackers and/or electromagnetic trackers.

The non-rotationally symmetric working end may have a substantially planar configuration (e.g., it may be configured as a cutting blade, a screw driver blade or a paddle-type manipulator). In other scenarios, the non-rotationally symmetric working end may have one or more protrusions that are angled-off a longitudinal axis of the working end (e.g., at an angle between 30° to 90° opening in a proximal direction, wherein the proximal direction faces towards the work object and the distal direction towards a tool user).

The first position data may have been acquired in a close temporal context with acquisition of the second position data. As an example, the first position data may have been acquired simultaneously with the second position data. In case the tool has a substantially fixed position during calibration (e.g., when being fixed in a tool holder), the first and second position data may be acquired at different points in time.

In some variants, the tool may be a surgical instrument with the first tool part being configured to be applied to a patient anatomy and the second tool part being a handle to be gripped by the surgeon. The working end of the first tool part may be configured for manipulation of tissue, for example for ablation of tissue, in particular for cutting or drilling of tissue, or for suction of tissue. In other variants, the tool may be a bone clamp, for example for vertebra tracking, with the working end of the first tool part comprising clamping brackets for attachment to the spinal process and the second tool part being configured to carry the tool tracker. The orientation of the working end may indicate the orientation of a bone, for example the vertebral process, the tool is attached to.

The first tool part and the second tool part may be configured so that the first part may be rotated relative to the second part (e.g., so that the two tool parts can assume multiple discrete or non-discrete angular orientations relative to each other). When a certain (e.g., user-selected) angular orientation is achieved, the first tool part may be releasably fixed relative to the second tool part in that angular orientation. In other implementations, the certain (e.g., undefined) angular orientation is the result of fixing the first tool part relative to the second tool part (e.g., using a wrench). Fixing the two tool parts to each other may be performed in a releasable or non-releasable manner.

In some implementations, at least one of the first and second position data may be indicative of one or more positions, orientations or poses of the tool tracker and the tracked pointer, respectively (e.g., of one or more positions and respective orientations). The first and second position data may be received from a data storage or directly from a tracking system or a navigation system with tracking capabilities (e.g., in case of optical tracking from a tracking camera that is part of a tracking or navigation system). The first and second position data may be received simultaneously or in any order.

In some variants, the reference structure comprises a planar surface. The planar surface may be provided in an otherwise curved (e.g., cylindrical) region of the first tool part. In such a case, the step of determining the angular orientation of the working end relative to the tool tracker may comprise determining, based on the second position data, an angular orientation defined by the planar surface. The angular orientation of the working end relative to the tool tracker may then be determined based on the first position data, the angular orientation of the planar surface and the predefined angular relation. The predefined angular relation may be pre-configured or read from a tool memory.

In some variants, the working end may be a substantially planar working end (e.g., a blade or having a paddle-like configuration) or a working end having a protrusion in an axial or radial direction relative to a longitudinal axis of the working end (e.g., the protrusion may serve for tissue manipulation). In some cases, the predefined angular relation may be indicative of an angular offset of the working end relative to the orientation of the planar surface comprised by the reference structure. This angular offset may be algorithmically considered by a corresponding offset parameter. In other cases, the predefined angular relation may be indicative of the working end having the same angular orientation as the planar surface comprised by the reference structure. In such a case, the resulting a priori knowledge may algorithmically be considered by setting the offset parameter to zero or omitting the offset parameter.

In certain implementations, the first tool part is substantially straight and defines a longitudinal axis. In other implementations, the first tool part comprises one or more bends (i.e., has a curved configuration). In such a case, at least a proximal portion of the first tool part carrying the working end may define a longitudinal axis that defines an angular offset relative to a distal portion of the first tool part. The angular offset may range between 5° and 90° (e.g., between 20° and 70°).

In some variants, the first tool part may be detachably attached to the second tool part. The first tool part may be attached to the second tool part via a screw connection or via a bayonet connection. The planar surface of the reference structure may be defined by a wrench flat configured to be engaged by a wrench to attach the first tool part to the second tool part by rotation of the first tool part relative to the second tool part.

In some variants, the first position data are indicative of at least one position of the tool tracker while the pointer is tracing the reference structure. The first position data may be indicative of different positions of the tool tracker while the pointer is tracing the reference structure. In such a case, the method may comprise compensating, when determining the angular orientation of the working end relative to the tool tracker, a movement of the tool tracker while the pointer is tracing the reference structure. For example, movement of the tool tracker may be detected during the tracing with the pointer and the pointer positions may be adapted based on the detected tool tracker movement (e.g., by subtracting the detected tool tracker movement).

The method may further comprise acquiring information indicative of an expected angular orientation, or expected orientation range, of the working end relative to the tool tracker, and determining a difference between the determined angular orientation and the expected angular orientation. In this manner, the method may comprise determining a clocking error. The information indicative of the expected angular orientation, or expected orientation range, may comprise at least one of manufacturing data and a digitized tool model.

The method may comprise outputting an error indication dependent on the difference between the determined angular orientation and the expected angular orientation. The indication may be an optical and or acoustical indication.

A screw connection between the first and second tool parts may be subject to a variable clocking error, e.g., due to different torques being applied via the wrench or due to wear of a thread of the screw connection. Performing the method described herein multiple times over the lifetime of a tool may be help to determine at least one of wear of a tool and the need to replace at least a part of the tool. Such information may then be output to a user.

In some variants, the reference structure of the tool comprises at least one region that does not constrain pointer movement in two perpendicular directions. As such, the pointer may be moved in a painting-like manner within that region.

The region of unconstrained pointer movement may be limited by one, two or more walls. The at least one wall may be provided on one, two or more sides (e.g., the region may entirely be surrounded by the at least one wall to constrain pointer movement to that region). The region of unconstrained pointer movement may be planar. The planar region may be provided in an otherwise curved (e.g., cylindrical) region of the first tool part. The reference structure, and in particular the region of unconstrained pointer movement, may have an oval (e.g., circular) or polygonal (e.g., rectangular, in particular quadratic) configuration. The reference structure, and in particular the region of unconstrained pointer movement, may have a size of at least 0.2, 0.5, 1 or 2 cm² and/or of less than 6, 4, 2 or 1 cm².

Tracing of the reference structure may be the result of a manual, in particular painting-like movement of the pointer. Alternatively, the tracing may be performed automatically (e.g., using a robotic arm).

The method may comprise receiving third position data indicative of a position of a trackable calibration device and one or more positions of the tool tracker when the tool is in contact with the trackable calibration device. In such a scenario, the method may further comprise determining tool calibration information based on the position data. The tool calibration information may be indicative of at least one of a position of a dedicated point of the working end relative to the tool tracker (e.g., relative to a reference point of the tool tracker such as an origin of a tracker coordinate system) and an axis of the tool.

In some variants, at least one of the tool tracker and the pointer is trackable by a tracking system. The tracking system may be an optical or an electromagnetic tracking system. The method may comprise determining, based on a tracking of at least one of the tool tracker and the pointer, at least one of a position, an orientation and a pose of the at least one of the tool tracker and the pointer within a coordinate system of the tracking system. When configured as an optical tracking system, the tracking system may comprise one or more tracking cameras and each tracker may comprise one or more optically active or optically passive (i.e., reflective) markers.

When the pose (i.e., position and orientation) of the tool tracker is determined within the coordinate system of the tracking system, the method may further comprise generating navigation instructions based at least in part on the determined pose of the tool tracker, the angular orientation of the working end relative to the tool tracker, and, optionally, the calibration information. The method may thus comprise calibrating the tool and navigating the tool, based on tracking of the pose of the tool tracker of the calibrated tool. Alternatively, the method may comprise navigating at least one other trackable tool based on the pose of the tool tracker, the angular orientation of the working end relative to the tool tracker and the calibration information. In some cases, the navigation instructions are configured to guide the working end relative a target site (e.g., a target anatomy of a patient).

According to a second aspect, a computer program product is provided. The computer program product comprises instructions that, when executed on at least one processor, cause the at least one processor to carry out any of the methods described herein.

According to a third aspect, an apparatus for determining an angular orientation between a non-rotationally symmetric working end of a first part of a tool and a tool tracker carried by a second part of the tool is provided. The first tool part is configured to be fixed to the second tool part in one of multiple angular orientations and comprises a reference structure. The reference structure is traceable by a tracked pointer and has a predefined angular relation to the working end. The apparatus comprises a processor configured to receive first position data indicative of one or more positions of the tool tracker, receive second position data indicative of pointer positions while the pointer is tracing the reference structure, and determine, based on the received first and second position data and the predefined angular relation between the reference structure and the working end, the angular orientation of the working end relative to the tool tracker.

The apparatus may be a computing device. The apparatus may be configured for receiving data from a tracking system.

The processor may be configured to perform any of the method steps described herein.

According to a fourth aspect, a system is provided. The system comprises the apparatus described herein. The system further comprises a tracking system configured to track at least one of the tool tracker and the pointer.

According to a variant, the system may further comprise the tool, wherein the reference structure is different from a wrench.

The reference structure may be a planar surface. The planar surface may laterally be limited by one or more walls configured to constrain lateral movement of the pointer.

### Brief Description of the Drawings

Further details, advantages and aspects of the present disclosure will become apparent from the following embodiments taken in conjunction with the drawings, wherein:
- Fig. 1A: shows a tool comprising a first tool part with a non-rotationally symmetric working end and a second tool part with a tool tracker carried by the second tool part;
- Fig. 1B: shows another tool similar to the first tool of Fig. 1A, with the working end having a first angular orientation relative to the tool tracker;
- Fig. 1C: shows the tool of Fig. 1B, with the working end having a second angular orientation relative to the tool tracker, different from the first angular orientation shown in Fig. 1B;
- Fig. 2: shows a system comprising a trackable pointer, a tracking camera and the tool of Fig. 1A;
- Fig. 3: shows the system of Fig. 2 with the pointer tracing the reference structure;
- Fig. 4: illustrates an acquisition of point and axis calibration information for a tool using a trackable calibration device;
- Fig. 5: Fig. 5 shows a flow diagram of a method for determining an angular orientation of a non-rotationally symmetric working end relative to a tool tracker;
- Fig. 6: shows a system comprising a tracking camera and a calibrated tool;
- Fig. 7A: shows a first tool part having an angled working end;
- Fig. 7B: shows the angled working end of Fig. 7A in detail;
- Fig. 7C: shows another tool comprising an angled first tool part;
- Fig. 8: shows a computer program product executable on at least one processor; and
- Fig. 9: shows a system comprising multiple tools, and a navigation system.

### Detailed Description

In the following description of exemplary embodiments, the same reference numerals are used to denote the same or similar structural components.

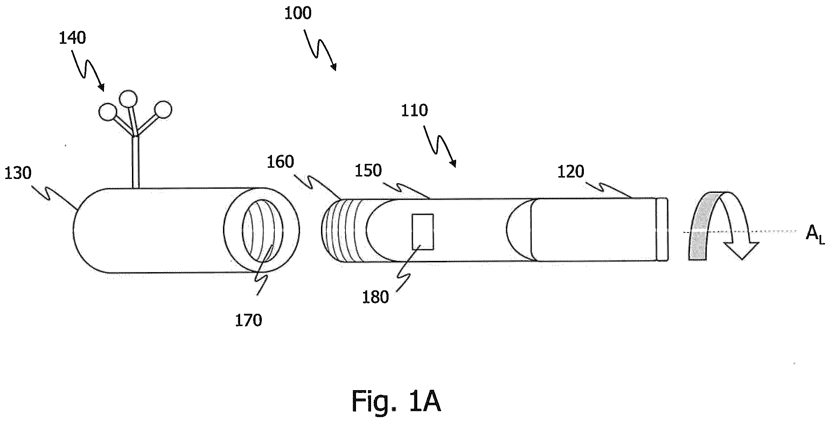

Fig. 1A shows an exemplary tool 100 that can be used in the context of embodiments of the present disclosure. The tool 100 comprises a first tool part 110 with a non-rotationally symmetric working end 120 (here: a flat blade) and a second tool part 130 with an optical tool tracker 140 carried by, and having a fixed angular and positional relation to, the second tool part 130. The first tool part 110 has a rotationally symmetric distal portion 150 defining a longitudinal axis A_{L}.

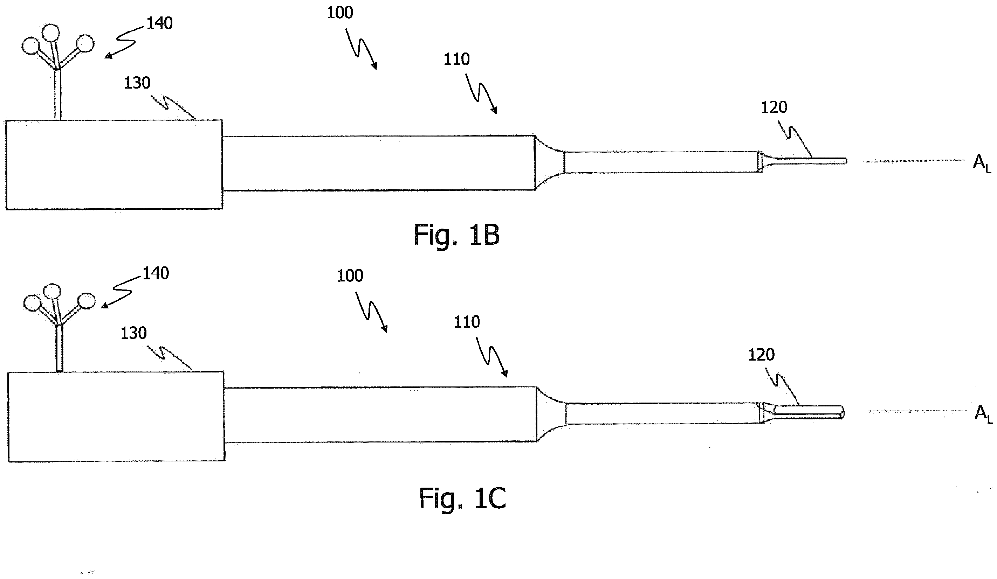

The tool tracker 140 may be detachable from the second tool part 130 or integrated into the second tool part 130. In the example shown in Fig. 1, the tool tracker 140 is an optical tracker. In more detail, the tool tracker 140 comprises multiple passive optical reflectors arranged in a predefined positional relationship relative to each other. In other cases, active optical markers (e.g., light emitting diodes) or an electromagnetic tool tracker (comprising, e.g., one or more coils) may be used.

As shown in Fig. 1A, one of the first and second tool parts 110, 130 comprises a thread 160, 170 and the other tool part 110, 130 comprises a corresponding counterthread 170, 160 configured to establish a screw connection between the two tool parts 110, 130. As a result, the first and second tool parts 110, 130 are detachably attachable to one another by a relative rotation around the axis A_{L}. Therefore, the second tool part 130 (e.g., a handle) may be used with different first tool parts 110 (e.g., different tissue manipulators) that can be exchanged by a user. For establishing a firm screw connection between the two tool parts 110, 130, the first tool part 110 is provided with an optional wrench flat 180 that is configured to be engaged by a wrench. The wrench flat 180 is provided in the otherwise cylindrical distal region of the first tool part 110.

In other implementations, the first and second tool parts 110, 130 may be detachably fixed to another via a bayonet connection. In still other implementations, the first and second tool parts 110, 130 may be fixable to one another in multiple (e.g., two or more) predefined, discrete angular orientations. In such or other implementations, the first and second tool parts 110, 130 may not necessarily be fully detachable from one another, but may be configured rotatable relative to one another (e.g., in a guided manner).

Fig. 1B shows a tool 100 similar to the tool 100 of Fig. 1A. The shown tool 100 comprises a first tool part 110 with a non-rotationally symmetric working end 120 (here: a paddle-like tissue manipulator) and a second tool part 130 carrying a tool tracker 140. The first and second tool parts 110, 130 are attached to one another. As illustrated in Fig. 1B, the working end 120 of the first tool part 110 has a first angular orientation relative to the tool tracker 140 with respect to a longitudinal axis A_{L}. Fig. 1C likewise shows the tool 100 of Fig. 1B with the first and second tool parts 110, 130 being attached to one another. As becomes apparent from Fig. 1C, the working end 120 has a second angular orientation relative to the tool tracker 140. The second angular orientation is different from the first angular orientation shown in Fig. 1B. Such a situation may result, for example, from wear or from different torques having been applied when attaching the first tool part 110 to the second tool part 130 in the scenarios of Fig. 1B and 1C (e.g., via a screw connection, see Fig. 1A).

Since the same angular orientation between the tool tracker 140 and the working end 120 was expected for the tool 100 shown in Figs. 1B and 1C, at least one of the shown configurations of the tool 100 is subject to a clocking error, i.e., a difference between an expected and the actual angular orientation. The clocking error may lead to a surgeon operating based on an erroneous orientation of the working end 120, when navigation information is determined based on tracking information indicative of a pose of the tool tracker 140. To avoid such an undesired case, the actual angular orientation has to be determined prior to generating navigation information.

In other scenarios, the angular orientation of the working end 120 relative to the second tool part 130 (i.e., the tool tracker 140) is selectable depending on the user's needs, possibly in one of multiple discrete angular positions. Therefore, the first tool part 110 and the second tool part 130 may be configured so that the first tool part 110 is rotatable relative to the second tool part 130, and when a desired angular orientation is achieved, the first tool part 110 can be releasably fixed relative to the second tool part 130 in that angular orientation.

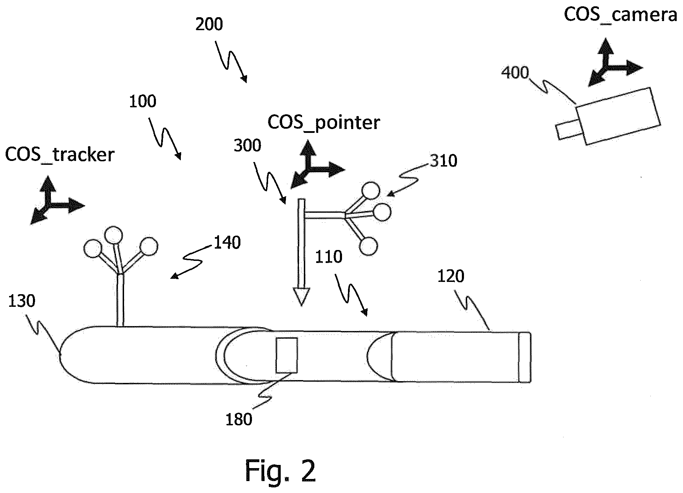

In all these scenarios, there is a need to determine (i.e., calibrate) the angular orientation between the non-rotationally symmetric working end 120 of the tool 100 and the tool tracker 140. To enable a determination of the actual angular orientation (e.g., the clocking error or the user-selected angular orientation), the tool 100 may be provided with a reference structure 180 as shown in Fig. 2. The reference structure 180 is a (here: rectangular) planar surface.

In some cases, the reference structure 180 may be defined by a wrench flat configured to be engaged by a wrench (not shown) to attach or detach the first tool part 110 to or from the second tool part 130. In other cases, the reference structure 180 may be a structure separate from a wrench flat so that wear of the wrench flat will not negatively influence calibration of the angular orientation. In still other cases, no wrench flat is provided at all (e.g., when a bayonet connection is implemented for the first and second tool parts 110, 130), but only the reference structure 180.

The reference structure 180 is configured to be engaged by the tip of a trackable pointer 300. The pointer 300 may be a common pointing device, for example a handheld pointer commonly used for surgical purposes. In the scenario of Fig. 2, the pointer 300 comprises a pointer tracker 310 similar to the tool tracker 140.

As illustrated in Fig. 2, a tracking camera 400 (e.g., a stereo camera) is provided for tracking at least one of a position and an orientation of the tool tracker 140 and a position and an orientation of the pointer tracker 310. The tracking camera 140 may be a part of a (e.g., surgical) tracking or navigation system. As further illustrated in Fig. 2, each of the tool tracker 140, the pointer tracker 310 and the camera 400 is associated with a dedicated coordinate system.

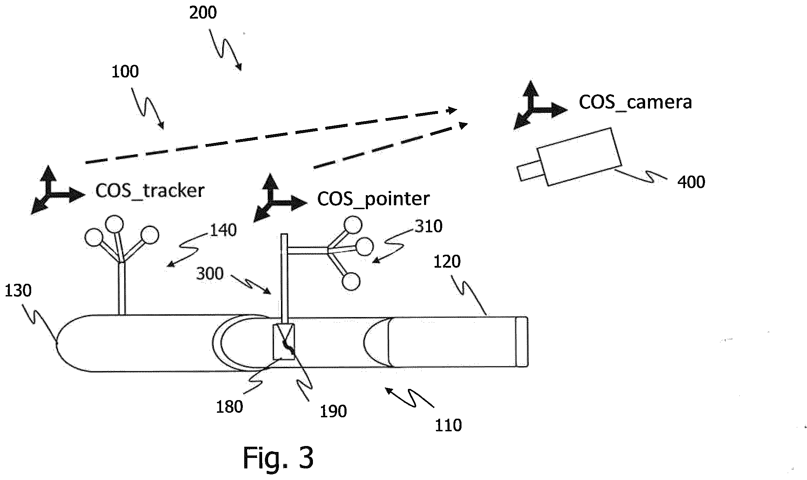

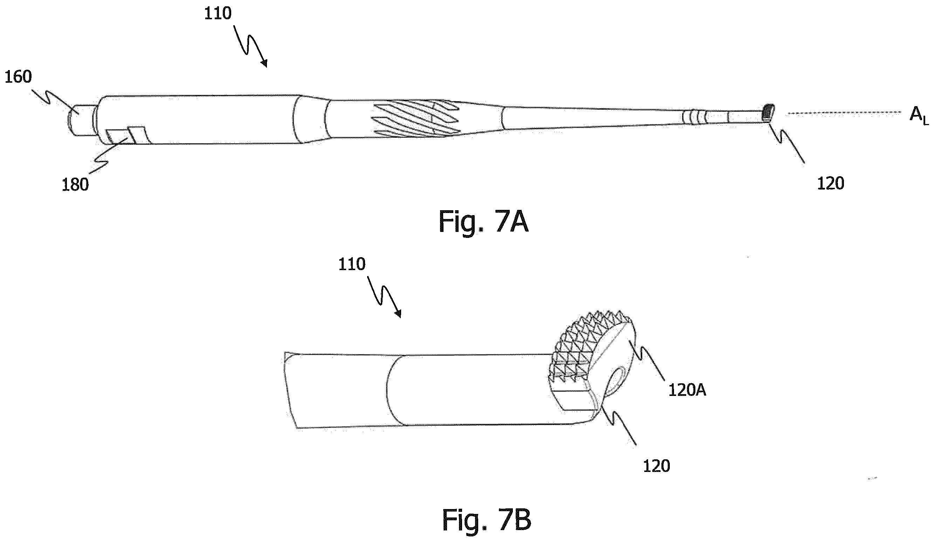

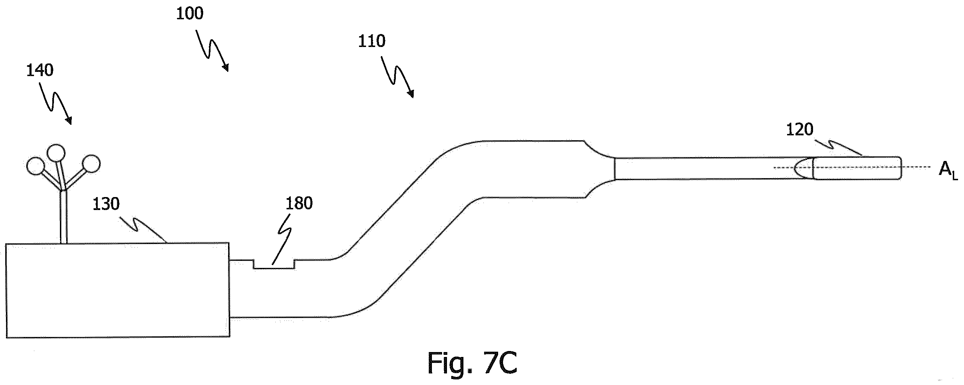

To determine the actual angular orientation between the non-rotationally symmetric working end 120 of the tool 100 and the tool tracker 140, the reference structure 180 is traced with the pointer 300. Such a tracing can, for example, result from a user manually moving the pointer 300 over the reference structure 180 in a painting-like and/or free-hand movement to define a trace 190, as shown in Fig. 3. This free-hand movement can occur within a certain region of the reference structure 180 and with two translatory degrees of freedom. The region may be defined by one, two ore more walls that laterally constrain a pointer movement. If, for example, the reference structure 180 is a wrench flat, the movement will typically be constrained by one or two parallel wrench flat walls running perpendicular to the longitudinal axis A_{L} (see Figs. 1A, 7A and 7C). In other cases, the reference structure 180 may fully be surrounded by a wall to limit pointer movement to the region of the reference structure 180. In still other cases, the reference structure 180 may be defined by one, two or more grooves or channels configured to define a planar reference surface and to guide pointer movement.

While tracing the reference structure 180, positions of the tool tracker 140 and the pointer 300 (i.e., the pointer tracker 310) are tracked by the tracking camera 140, as indicated by respective dashed arrows in Fig. 3. In the shown example, the tool tracker 140 and the pointer 300 are tracked by the same camera 400. The positions of the tool tracker 140 and the pointer 300, i.e., a tip of the pointer 300, are determined in the same coordinate system, preferably the camera coordinate system COS_camera. The camera coordinate system COS_camera may define a global coordinate system for use by a tracking and/or navigation system. Alternatively, the coordinates may be transformed into any other suitable coordinate system, e.g., a coordinate system defined by the tool tracker 140 or the pointer 300. The coordinates of the tip of the pointer 300 may be determined based on a known relation between the pointer tracker 310 attached to the pointer 300 and the tip of the pointer 300. Further, a possible movement of the tool tracker 140 during the tracing operation is taken into account for determining the pointer positions. In particular, an algorithm for determining the pointer positions may compensate the tracked positions of the pointer tracker 310 based on the tracked movement of the tool tracker 140. As an example, any tool tracker movement during the tracing operation may be subtracted from the tracked positions of the pointer tracker 310.

Based on the determined pointer positions, an orientation of the reference structure 180 within the common coordinate system (e.g., COS_camera) can be determined. This orientation may take the geometric form of a plane or a portion of the plane within that coordinate system. Further, based on a known angular relation of the reference structure 180 to the working end 120, an orientation of the working end 120 in the common coordinate system can be determined. Since one or more tool tracker positions or poses are also (e.g., concurrently) determined in the common coordinate system, an angular relation of the working end 120 relative to the tool tracker 140 can be determined as angular calibration information.

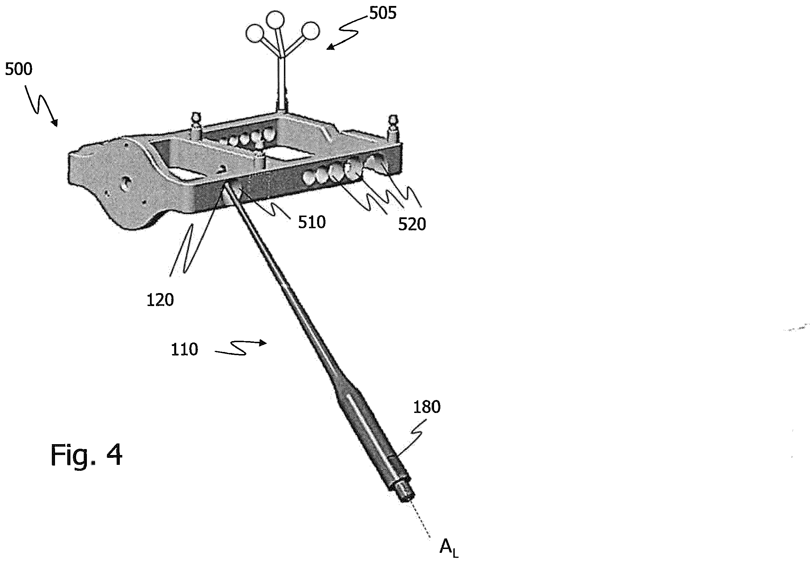

For optionally acquiring additional calibration information, e.g., one or both of a relative position of the working end 120 (e.g., of a dedicated point thereof) to the tool tracker 140 and the orientation of a longitudinal axis A_{L} defined by at least a portion of the tool 100, a trackable calibration device 500 may be used as shown in Fig. 4. The calibration device 500 comprises a device tracker 505 similar to the tool tracker 140. The device tracker 505 is configured to be tracked by the tracking camera 400.

As illustrated in Fig. 4, a dedicated and predefined point of the working end 120 may be brought into contact with a reference structure 510 of the calibration device 500 (e.g., a dimple or cone-shaped opening as shown in Fig. 4). The position of the predefined point of the working end 120 relative to the tool tracker 140 may then be determined based on a known relation between the reference structure 510 of the calibration device 500 and the device tracker 505 on the one hand and relative positions of the tool tracker 140 and the device tracker 505 on the other hand plus, optionally, further information. Analogously, the orientation of the longitudinal axis A_{L} of at least a portion of the tool 100 may be determined when that portion of the tool 100 is received by another reference structure 520 of the calibration device 500. In the example of Fig. 4, this other reference structure 520 comprises pairs of concentric holes arranged on opposite sides of a frame of the calibration device 500. By fittingly inserting a portion of the tool 100 into a pair of holes and then rotating that portion of the tool 100 relative to the holes while tracking the tool tracker 140 (not shown in Fig. 4) and possibly the device tracker 505 to compensate for any movements of the calibration device 500, the orientation of the longitudinal axis A_{L} can be determined.

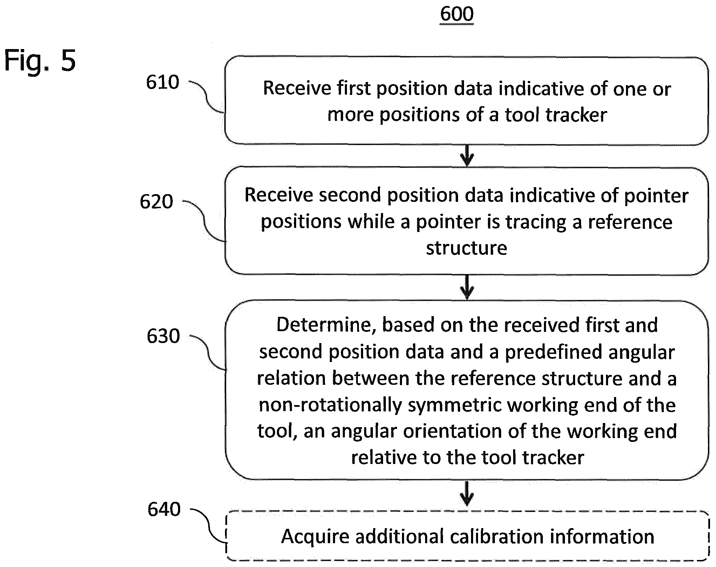

Fig. 5 shows a flow diagram of a method 600 for determining an angular orientation of a non-rotationally symmetric working end 120 relative to a tool tracker 140 that is based on the above-described scenarios or other scenarios. The method 600 may be used for determining the angular orientation of the non-rotationally symmetric working ends 120 relative to a tool trackers 140 of any of the tools 100 described herein.

In a first and a second step 610, 620 the method 600 comprises receiving first and second position data. The first position data is indictive of at least one or more positions of a tool tracker 140. The first position data may also be indicative of one or more poses of the tool tracker 140. The second position data is indicative of pointer positions while the pointer 300 is tracing a reference structure 180.

In a third step 630, the angular orientation of the working end 120 relative to the tool tracker 140 is determined based on the received first and second position data and a predefined angular relation between the reference structure 180 and the non-rotationally symmetric working end 120 of the tool 100, as described with reference to Figs. 2 to 3.

In some variants, a difference between the angular orientation determined in step 630 and an expected angular orientation or orientation range is determined. In this manner, a clocking error can be detected. The information indicative of the expected angular orientation, or expected orientation range, may be derived from at least one of manufacturing data and a digitized tool model. An error indication may then be output dependent on the difference between the determined angular orientation and the expected angular orientation. The indication may be an optical and or acoustical indication. The indication may vary based on the amount of the difference. For example, a colour, type or volume of the indication may be adapted based on the amount of the difference.

In an optional further step 640 that may be performed independently from steps 610 to 630, additional calibration information is acquired. The additional calibration information may include one or both of a point calibration and an axis calibration as discussed above with reference to Fig. 4.

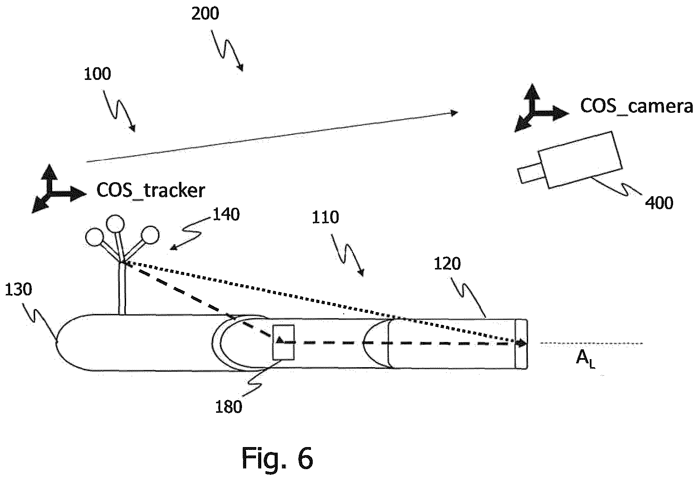

Fig. 6 shows a system comprising the tracking camera 400 and the calibrated tool 100 after the steps 610 to 640 of Fig. 5 have been performed. As schematically described with reference to Figs. 2 to 5, the tool 100 has been calibrated to determine the angular orientation of the working end 120 relative to the tool tracker 140 (dashed arrows) and one or both of the relative position of a predefined point of the working end 120 (e.g., a blade edge center) to the tool tracker 140 (dotted arrow) and the orientation of the longitudinal axis A_{L} of the tool 100.

During a surgical procedure, the tool tracker 140 of the calibrated tool 100 (e.g., an origin of the tracker coordinate system COS_tracker) can then tracked by the tracking camera 400 in the camera coordinate system COS_camera. A computing system coupled to the tracking camera 400 will track the position and orientation of the tool tracker 140 and, based on the calibration information, calculate in the camera coordinate system COS_camera the orientation of the working end 120 and, optionally, one or both of the position of the predefined point of the working end 120 and the orientation of the longitudinal axis A_{L} of the tool 100. The resulting information may be used for generating navigation instructions. In some cases, the navigation instructions are visualized on a display as a current position and orientation of the working end 120 relative to a patient image. In other cases, the navigation instructions are used to control a surgical robot.

Figs. 7A to 7C show alternative variants for tools 100 with non-rotationally symmetric working ends 120.

Figs. 7A and 7B show a first tool portion 110 with a working end 120 that that has an angled protrusion 120A relative to the longitudinal axis A_{L} of the tool 100. In the present example, a radially outer surface of the protrusion 120A has a rasp-like surface configured to engage an object, e.g., tissue of a patient. In this or other variants, the first tool portion 110 may be hollow to define a suction channel. A corresponding suction source may be attached to the second tool portion 120 not illustrated in Figs. 7A and 7B. The protrusion 120A may generally be configured to manipulate (e.g., move or remove) tissue in the context of the suction procedure.

The thread 160 for attaching the first tool part 110 to a handle or other second tool part 120 is illustrated only schematically in Fig. 7A (as a cylindrical surface). In the example of Fig. 7A, the reference structure 180 is defined by a stepped profile resulting from two immediately adjacent wrench flats to enable engagement by differently sized wrenches.

Fig. 7C shows a tool 100 similar to the tools shown in Figs. 2 to 5, wherein the first tool part 110 is angled in a Z-like manner. The longitudinal axis A_{L} is defined by an extension of the proximal portion of the second tool part 120. Due to the lateral offset of the second tool part 130 from the longitudinal axis A_{L}, any clocking error will be amplified in the tracking procedure, making a correct calibration even more important.

In the example of Fig. 7C, the reference structure 180 is a single wrench flat that comprises two parallel walls. These walls laterally limit a plane extending perpendicularly to a plane defined by the flat working end 120. As such, the predefined angular orientation between the reference structure 180 and the working end 120 is 90°, while in other examples it could be 0° or any other angle.

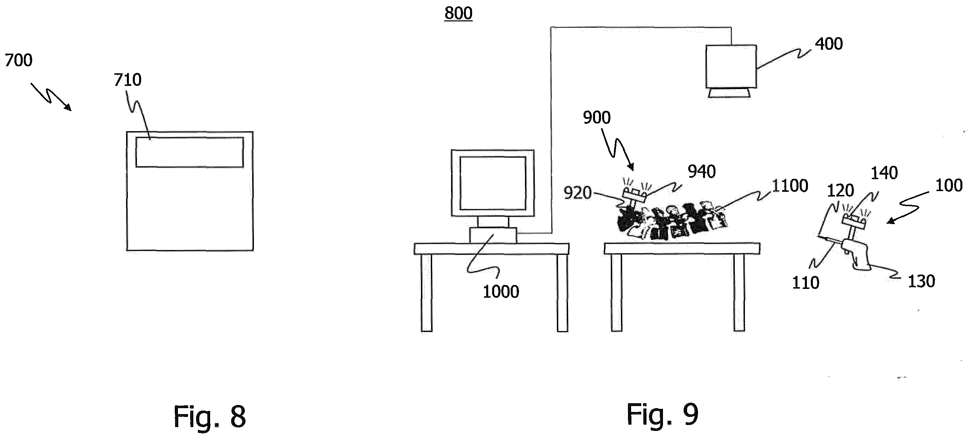

Fig. 8 shows a computer program product 700 comprising instructions 710 that, when executed on at least one processor, cause the at least one processor to carry out any of the methods described herein. The processor may be part of a local or cloud-based computing device.

Fig. 9 shows a surgical system 800 comprising multiple tools 100, 900, a tracking camera 400 and an apparatus 1000 configured to perform the method steps described herein. The apparatus 1000 may be a computing device operated on the basis of the computer program product 700 and, in the present example, also comprises a display for outputting graphical instructions in a surgical navigation context.

The first tool is a tool 100 similar to the ones described above, e.g., an instrument that may be held and operated by a surgeon (e.g., a surgical power tool or a surgical suction device). The second tool 900 is a bone clamp, e.g., a vertebra clamp, with a non-rotationally symmetric working end 920 (e.g., clamping brackets, not shown) for clamping the tool 900 to a vertebra of a patient 1100. The second tool 900 is configured to carry a bone tracker 940 in one of multiple angular relationships relative to the working end 920. The clamping brackets 920 and bone tracker 940 are carried by tool parts that can be angled and fixed relative to each other in a user-selectable manner. The tool parts cannot be separated from each other, but allow a relative rotation to optimize visibility of the bone tracker 940 for the tracking camera 400.

The tracking camera 400 is configured track one or both of the tools 100, 900. The apparatus 1000 is configured generate and visualize navigation instructions for navigating the first tool 100 relative to at least one of the patient, the second tool 900, further trackers, and further tools (not shown). Additionally or alternatively, the apparatus 1000 may generate navigation information for navigating another tool (not shown) relative to at least one of the second tool 900, further trackers, and further tools (not shown).

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A method (600) for determining an angular orientation between a non-rotationally symmetric working end (120, 920) of a first part (110) of a tool (100, 900) and a tool tracker (140) carried by a second part (130) of the tool (100, 900), wherein the first tool part (110) is configured to be fixed to the second tool part (130) in one of multiple angular orientations and comprises a reference structure (180), wherein the reference structure (180) is traceable by a tracked pointer (300) and has a predefined angular relation to the working end (120, 920), the method (600) comprising:
receiving (610) first position data indicative of one or more positions of the tool tracker (140);
receiving (620) second position data indicative of pointer positions while the pointer (300) is tracing the reference structure (180); and
determining (630), based on the received first and second position data and the predefined angular relation between the reference structure (180) and the working end (120, 920), the angular orientation of the working end (120, 920) relative to the tool tracker (140).

2. The method (600) according to claim 1, wherein
the reference structure (180) comprises a planar surface.

3. The method (600) according to claim 2, wherein
the predefined angular relation is indicative of the working end (120, 920) having the same angular orientation as the planar surface comprised by the reference structure (180).

4. The method according to any preceding claim, wherein
the first tool part (110) is detachably attached to the second tool part (130), optionally via a screw connection (150, 160).

5. The method (600) according to any of claims 2 and 3 or claim 4, when depending on claim 2 or 3, wherein
the planar surface is defined by a wrench flat configured to be engaged by a wrench to attach the first tool part (110) to the second tool part (130).

6. The method (600) according to any preceding claim, wherein
the first position data are indicative of at least one position of the tool tracker (140) while the pointer (300) is tracing the reference structure (180).

7. The method (600) according to any preceding claim, wherein
the first position data are indicative of different positions of the tool tracker (140) while the pointer (300) is tracing the reference structure (180), and comprising:
compensating, when determining the angular orientation of the working end (120, 920) relative to the tool tracker (140), a movement of the tool tracker (140) while the pointer (130) is tracing the reference structure (180).

8. The method (600) according to any preceding claim, further comprising:
acquiring information indicative of an expected angular orientation, or expected orientation range, of the working end (120, 920) relative to the tool tracker (140); and
determining a difference between the determined angular orientation and the expected angular orientation.

9. The method (600) according to claim 8, comprising
outputting an error indication dependent on the difference between the determined angular orientation and the expected angular orientation.

10. The method (600) according to any preceding claim, wherein
the reference structure (180) comprises at least one region that does not constrain pointer movement in two perpendicular directions.

11. The method (600) according to any preceding claim, wherein
tracing of the reference structure (180) is the result of a manual movement of the pointer (300).

12. The method (600) according to any preceding claim, further comprising:
receiving third position data indicative of a position of a trackable calibration device (500) and one or more positions of the tool tracker (140) when the tool (100, 900) is in contact with the trackable calibration device (500); and
determining tool calibration information based on the third position data.

13. The method according to any preceding claim, wherein at least one of the tool tracker (140) and the pointer (300) is trackable by a tracking system, the method (600) further comprising:
determining, based on a tracking of at least one of the tool tracker (140) and the pointer (130), at least one of a position, orientation or a pose of the at least one of the tool tracker (140) and the pointer (130) within a coordinate system of the tracking system.

14. The method (600) according to claims 12 and 13, further comprising:
when the pose of the tool tracker (140) is determined within the coordinate system of the tracking system, generating navigation instructions based at least in part on the determined pose of the tool tracker (140), the angular orientation of the working end (120, 920) relative to the tool tracker (140) and the calibration information, wherein the navigation instructions are configured to guide the working end (120, 920) relative a target site.

15. A computer program product (700), comprising instructions (710) that, when executed on at least one processor, cause the at least one processor to carry out any of the methods of claims 1 to 14.

16. An apparatus (1000)
for determining an angular orientation between a non-rotationally symmetric working end (120, 920) of a first part (110) of a tool (100, 900) and a tool tracker (140) carried by a second part (130) of the tool (100, 900), wherein the first tool part (110) is configured to be fixed to the second tool part (130) in one of multiple angular orientations and comprises a reference structure (180), wherein the reference structure (180) is traceable by a tracked pointer (300) and has a predefined angular relation to the working end (120, 920), the apparatus comprising a processor configured to:
receive (610) first position data indicative of one or more positions of the tool tracker (140);
receive (620) second position data indicative of pointer positions while the pointer (300) is tracing the reference structure (180); and
determine (630), based on the received first and second position data and the predefined angular relation between the reference structure (180) and the working end (120, 920), the angular orientation of the working end (120, 920) relative to the tool tracker (140).

17. The apparatus (1000) of claim 16, wherein the processor is configured to perform any of the method steps of claims 2 to 14.

18. A system (800) comprising:
the apparatus (1000) according to any one of claims 16 and 17; and
a tracking system configured to track at least one of the tool tracker (140) and the pointer (300).

19. The system according to claim 18, when not depending on claim 5, further comprising:
the tool (100, 900), wherein the reference structure (180) is different from a wrench flat.

20. The system according to claim 19, wherein
the reference structure is a planar surface which, as an option, is laterally limited by one or more walls configured to constrain lateral movement of the pointer (300).

## Patentansprüche

1. Verfahren (600) zum Bestimmen einer Winkelausrichtung zwischen einem nicht rotationssymmetrischen Arbeitsende (120, 920) eines ersten Teils (110) eines Werkzeugs (100, 900) und einem Werkzeugtracker (140), der von einem zweiten Teil (130) des Werkzeugs (100, 900) getragen wird, wobei das erste Werkzeugteil (110) dazu eingerichtet ist, in einer von mehreren Winkelausrichtungen am zweiten Werkzeugteil (130) befestigt zu werden, und eine Referenzstruktur (180) umfasst, wobei die Referenzstruktur (180) von einem verfolgten Zeiger (300) abfahrbar ist und eine vordefinierte Winkelbeziehung zum Arbeitsende (120, 920) aufweist, wobei das Verfahren (600) Folgendes umfasst:
Empfangen (610) erster Positionsdaten, die eine oder mehrere Positionen des Werkzeugtrackers (140) angeben;
Empfangen (620) zweiter Positionsdaten, die Zeigerpositionen angeben, während der Zeiger (300) die Referenzstruktur (180) abfährt; und
Bestimmen (630) der Winkelausrichtung des Arbeitsendes (120, 920) relativ zum Werkzeugtracker (140) basierend auf den empfangenen ersten und zweiten Positionsdaten und der vordefinierten Winkelbeziehung zwischen der Referenzstruktur (180) und dem Arbeitsende (120, 920).

2. Verfahren (600) nach Anspruch 1, wobei
die Referenzstruktur (180) eine ebene Oberfläche umfasst.

3. Verfahren (600) nach Anspruch 2, wobei
die vordefinierte Winkelbeziehung angibt, dass das Arbeitsende (120, 920) dieselbe Winkelausrichtung aufweist wie die von der Referenzstruktur (180) umfasste ebene Oberfläche.

4. Verfahren nach einem der vorhergehenden Ansprüche, wobei
das erste Werkzeugteil (110) lösbar am zweiten Werkzeugteil (130) befestigt ist, optional über eine Schraubverbindung (150, 160).

5. Verfahren (600) nach einem der Ansprüche 2 und 3 oder Anspruch 4, sofern dieser von Anspruch 2 oder 3 abhängt, wobei
die ebene Oberfläche durch eine Schlüsselfläche definiert ist, die dazu eingerichtet ist, mit einem Schraubenschlüssel in Eingriff gebracht zu werden, um das erste Werkzeugteil (110) am zweiten Werkzeugteil (130) zu befestigen.

6. Verfahren (600) nach einem der vorhergehenden Ansprüche, wobei
die ersten Positionsdaten mindestens eine Position des Werkzeugtrackers (140) angeben, während der Zeiger (300) die Referenzstruktur (180) abfährt.

7. Verfahren (600) nach einem der vorhergehenden Ansprüche, wobei
die ersten Positionsdaten unterschiedliche Positionen des Werkzeugtrackers (140) angeben, während der Zeiger (300) die Referenzstruktur (180) abfährt, und umfassend:
Kompensieren, beim Bestimmen der Winkelausrichtung des Arbeitsendes (120, 920) relativ zum Werkzeugtracker (140), einer Bewegung des Werkzeugtrackers (140) während der Zeiger (130) die Referenzstruktur (180) abfährt.

8. Verfahren (600) nach einem der vorhergehenden Ansprüche, ferner umfassend:
Beschaffen von Informationen, die eine erwartete Winkelausrichtung oder einen erwarteten Ausrichtungsbereich des Arbeitsendes (120, 920) relativ zum Werkzeugtracker (140) angeben; und
Bestimmen einer Differenz zwischen der bestimmten Winkelausrichtung und der erwarteten Winkelausrichtung.

9. Verfahren (600) nach Anspruch 8, umfassend
Ausgeben eines Fehlerhinweise abhängig von der Differenz zwischen der bestimmten Winkelausrichtung und der erwarteten Winkelausrichtung.

10. Verfahren (600) nach einem der vorhergehenden Ansprüche, wobei
die Referenzstruktur (180) mindestens einen Bereich umfasst, der eine Zeigerbewegung in zwei senkrecht zueinander stehenden Richtungen nicht einschränkt.

11. Verfahren (600) nach einem der vorhergehenden Ansprüche, wobei
das Abfahren der Referenzstruktur (180) das Ergebnis einer manuellen Bewegung des Zeigers (300) ist.

12. Verfahren (600) nach einem der vorhergehenden Ansprüche, ferner umfassend:
Empfangen von dritten Positionsdaten, die eine Position einer verfolgbaren Kalibriervorrichtung (500) und eine oder mehrere Positionen des Werkzeugtrackers (140) angeben, wenn das Werkzeug (100, 900) mit der verfolgbaren Kalibriervorrichtung (500) in Kontakt ist; und
Bestimmen von Werkzeugkalibrierungsinformationen basierend auf den dritten Positionsdaten.

13. Verfahren nach einem der vorhergehenden Ansprüche, wobei der Werkzeugtracker (140) und/oder der Zeiger (300) durch ein Verfolgungssystem verfolgbar ist, wobei das Verfahren (600) ferner umfasst:
Bestimmen, basierend auf einer Verfolgung des Werkzeugtrackers (140) und/oder des Zeigers (130), mindestens einer von einer Position, einer Ausrichtung oder einer Pose des Werkzeugtrackers (140) und/oder des Zeigers (130) innerhalb eines Koordinatensystems des Verfolgungssystems.

14. Verfahren (600) nach den Ansprüchen 12 und 13, ferner umfassend:
wenn die Pose des Werkzeugtrackers (140) innerhalb des Koordinatensystems des Verfolgungssystems bestimmt wird, Erzeugen von Navigationsanweisungen, die mindestens teilweise auf der bestimmten Pose des Werkzeugtrackers (140), der Winkelausrichtung des Arbeitsendes (120, 920) relativ zum Werkzeugtracker (140) und den Kalibrierungsinformationen basieren, wobei die Navigationsanweisungen so konfiguriert sind, dass sie das Arbeitsende (120, 920) relativ zu einer Zielstelle führen.

15. Computerprogrammprodukt (700), das Anweisungen (710) umfasst, die, wenn sie auf mindestens einem Prozessor ausgeführt werden, den mindestens einen Prozessor veranlassen, eines der Verfahren der Ansprüche 1 bis 14 auszuführen.

16. Vorrichtung (1000) zum Bestimmen einer Winkelausrichtung zwischen einem nicht rotationssymmetrischen Arbeitsende (120, 920) eines ersten Teils (110) eines Werkzeugs (100, 900) und einem Werkzeugtracker (140), der von einem zweiten Teil (130) des Werkzeugs (100, 900) getragen wird, wobei das erste Werkzeugteil (110) dazu eingerichtet ist, in einer von mehreren Winkelausrichtungen am zweiten Werkzeugteil (130) befestigt zu werden, und eine Referenzstruktur (180) umfasst, wobei die Referenzstruktur (180) von einem verfolgten Zeiger (300) abfahrbar ist und eine vordefinierte Winkelbeziehung zum Arbeitsende (120, 920) aufweist, wobei die Vorrichtung einen Prozessor umfasst, der eingerichtet ist zum:
Empfangen (610) erster Positionsdaten, die eine oder mehrere Positionen des Werkzeugtrackers (140) angeben;
Empfangen (620) zweiter Positionsdaten, die Zeigerpositionen angeben, während der Zeiger (300) die Referenzstruktur (180) abfährt; und
Bestimmen (630) der Winkelausrichtung des Arbeitsendes (120, 920) relativ zum Werkzeugtracker (140) basierend auf den empfangenen ersten und zweiten Positionsdaten und der vordefinierten Winkelbeziehung zwischen der Referenzstruktur (180) und dem Arbeitsende (120, 920).

17. Vorrichtung (1000) nach Anspruch 16, wobei der Prozessor dazu eingerichtet ist, beliebige der Verfahrensschritte der Ansprüche 2 bis 14 auszuführen.

18. System (800), umfassend:
die Vorrichtung (1000) gemäß einem der Ansprüche 16 und 17; und
ein Verfolgungssystem, das dazu eingerichtet ist, den Werkzeugtracker (140) und/oder den Zeiger (300) zu verfolgen.

19. System nach Anspruch 18, sofern es nicht von Anspruch 5 abhängt, ferner umfassend:
das Werkzeug (100, 900), wobei die Referenzstruktur (180) von einer Schlüsselfläche verschieden ist.

20. System nach Anspruch 19, wobei
die Referenzstruktur eine ebene Oberfläche ist, die optional seitlich durch eine oder mehrere Wände begrenzt ist, die dazu eingerichtet sind, eine seitliche Bewegung des Zeigers (300) einzuschränken.

## Revendications

1. Procédé (600) pour déterminer une orientation angulaire entre un embout de travail non symétrique en rotation (120, 920) d'une première partie (110) d'un outil (100, 900) et un dispositif de suivi d'outil (140) porté par une deuxième partie (130) de l'outil (100, 900), dans lequel la première partie d'outil (110) est conçue pour se fixer à la deuxième partie d'outil (130) dans l'une parmi de multiples orientations angulaires et comprend une structure de référence (180), la structure de référence (180) pouvant être suivie par un pointeur suivi (300) et a une relation angulaire prédéfinie avec l'embout de travail (120, 920), le procédé (600) comprenant:
la réception (610) de premières données de position indiquant une ou plusieurs positions du dispositif de suivi d'outil (140);
la réception (610) de deuxièmes données de position indiquant les positions du pointeur (300) qui suit la structure de référence (8); et
la détermination (630), sur la base des première et deuxième données de position reçues et de la relation angulaire prédéfinie entre la structure de référence (180) et l'embout de travail (120, 920), de l'orientation angulaire de l'embout de travail (120, 920) par rapport au dispositif de suivi d'outil (140).

2. Procédé (600) selon la revendication 1, dans lequel
la structure de référence (180) comprend une surface plane.

3. Procédé (600) selon la revendication 2, dans lequel
la relation angulaire prédéfinie indique que l'embout de travail (120, 920) a la même orientation angulaire que la surface plane formée par la structure de référence (180).

4. Procédé selon l'une quelconque revendication précédente, dans lequel
la première partie d'outil (110) est fixée de manière amovible à la deuxième partie d'outil (130), éventuellement par l'intermédiaire d'un raccord vissé (150, 160).

5. Procédé (600) selon l'une quelconque des revendications 2 et 3 ou la revendication 4, lorsqu'elle dépend de la revendication 2 ou 3, dans lequel
la surface plane est définie par un pan de manœuvre conçue pour venir en prise avec une clé afin de fixer la première partie d'outil (110) à la deuxième partie d'outil (130).

6. Procédé (600) l'une quelconque revendication précédente, dans lequel
les premières données de position indiquent au moins une position du dispositif de suivi d'outil (140) pendant que le pointeur (300) suit la structure de référence (180).

7. Procédé (600) l'une quelconque revendication précédente, dans lequel
les premières données de position indiquent différentes positions du dispositif de suivi d'outil (140) pendant que le pointeur (300) suit la structure de référence (180), et comprenant:
la compensation, lors de la détermination de l'orientation angulaire de l'embout de travail (120, 920) par rapport au dispositif de suivi d'outil (140), d'un mouvement du dispositif de suivi d'outil (140) pendant que le pointeur (130) suit la structure de référence (180).

8. Procédé (600) selon l'une quelconque revendication précédente, comprenant en outre:
l'acquisition des informations indiquant une orientation angulaire attendue, ou une plage d'orientation attendue, de l'embout de travail (120, 920) par rapport au dispositif de suivi d'outil (140); et
la détermination d'une différence entre l'orientation angulaire déterminée et l'orientation angulaire attendue.

9. Procédé (600) selon la revendication 8, comprenant
l'indication d'erreurs en fonction de la différence entre l'orientation angulaire déterminée et l'orientation angulaire attendue.

10. Procédé (600) l'une quelconque revendication précédente, dans lequel
la structure de référence (180) comprend au moins une région qui ne limite pas le mouvement du pointeur dans deux directions perpendiculaires.

11. Procédé (600) l'une quelconque revendication précédente, dans lequel
le tracé de la structure de référence (180) est le résultat d'un mouvement manuel du pointeur (300).

12. Procédé (600) selon l'une quelconque revendication précédente, comprenant en outre:
la réception de troisièmes données de position indiquant une position d'un dispositif d'étalonnage détectable (500) et une ou plusieurs positions du dispositif de suivi d'outil (140) lorsque l'outil (100, 900) est en contact avec le dispositif d'étalonnage détectable (500); et
la détermination d'informations d'étalonnage de l'outil sur la base des troisièmes données de position.

13. Procédé selon l'une quelconque revendication précédente, dans lequel au moins l'un parmi le dispositif de suivi d'outil (140) et le pointeur (300) est détectable par un système de suivi, le procédé (600) comprenant en outre
la détermination, sur la base d'un suivi d'au moins l'un parmi le dispositif de suivi d'outil (140) et le pointeur (130), d'au moins l'une parmi une position, une orientation ou une pose de l'au moins un parmi le dispositif de suivi d'outil (140) et le pointeur (130) dans un système de coordonnées du système de suivi.

14. Procédé (600) selon les revendication 12 et 13, comprenant en outre:
lorsque la mise en place du dispositif de suivi d'outil (140) est déterminée dans le système de coordonnées du système de suivi, la génération d'instructions de navigation basées au moins en partie sur la mise en place déterminée du dispositif de suivi d'outil (140), de l'orientation angulaire de l'embout de travail (120, 920) par rapport au dispositif de suivi d'outil (140) et des informations d'étalonnage, les instructions de navigation permettant de guider l'embout de travail (120, 920) par rapport à un site cible.

15. Produit programme informatique (700), comprenant des instructions (710) qui, lorsqu'elles sont exécutées sur au moins un processeur, amènent l'au moins un processeur à mettre en œuvre l'un quelconque des procédés des revendications 1 à 14.

16. Appareil (1000)
permettant de déterminer une orientation angulaire entre un embout de travail non symétrique en rotation (120, 920) d'une première partie (110) d'un outil (100, 900) et un dispositif de suivi d'outil (140) porté par une deuxième partie (130) de l'outil (100, 900), dans lequel la première partie d'outil (110) est conçue pour se fixer à la deuxième partie d'outil (130) dans l'une parmi de multiples orientations angulaires et comprend une structure de référence (180), la structure de référence (180) pouvant être suivie par un pointeur suivi (300) et a une relation angulaire prédéfinie avec l'embout de travail (120, 920), l'appareil comprenant un processeur configuré pour:
recevoir (610) les premières données de position indiquant une ou plusieurs positions du dispositif de suivi d'outil (140);
recevoir les deuxièmes données de position indiquant les positions du pointeur (300) qui suit la structure de référence (8); et
déterminer (630), sur la base des première et deuxième données de position reçues et de la relation angulaire prédéfinie entre la structure de référence (180) et l'embout de travail (120, 920), de l'orientation angulaire de l'embout de travail (120, 920) par rapport au dispositif de suivi d'outil (140).

17. Appareil (1000) selon la revendication 16, dans lequel le processeur est configuré pour effectuer l'une quelconque des étapes du procédé selon les revendications 2 à 14.

18. Système (800) comprenant:
l'appareil selon l'une quelconque des revendications 16 et 17; et
un système de suivi configuré pour suivre au moins l'un parmi le dispositif de suivi d'outil (140) et le pointeur (300).

19. Système selon la revendication 18, lorsqu'elle ne dépend pas de la revendication 5, comprenant en outre:
l'outil (100, 900), la structure de référence (180) étant différente d'un pan de manœuvre.

20. Système selon la revendication 19, dans lequel
la structure de référence est une surface plane qui, éventuellement, est limitée latéralement par une ou plusieurs parois conçues pour limiter le mouvement latéral du pointeur (300).
